# EUROPEAN PATENT APPLICATION

(11) **EP 4 545 554 A1**
(43) Date of publication of application: **30.04.2025**
(21) Application number: 23205682.0
(22) Date of filing: 25.10.2023
(51) Int. Cl.: C07K 14/315, C07K 1/22, C12N 15/62

(54) **A RECOMBINANT POLYPEPTIDE COMPRISING A METAL-BINDING MOTIF**

(71) Applicant: Hober Biotech AB, 115 46 Stockholm (SE)
(72) Inventor: HOBER, Sophia, 115 46 Stockholm (SE); JÖNSSON, Malin, 112 48 Stockholm (SE)
(74) Representative: Kransell & Wennborg KB

(57) **Abstract**

The present disclosure generally relates to a recombinant polypeptide comprising an IgG-binding domain derived from *Streptococcus* Protein G (SpG) and a metal-binding motif, wherein the recombinant polypeptide has a first, target-binding configuration in the presence of metal ions, and a second, non-target-binding configuration in the absence of metal ions, wherein the recombinant polypeptide is configured to bind to the Fab-region of IgG in the first, target-binding configuration.

The present disclosure also relates to a ligand comprising the recombinant polypeptide, a chromatography matrix comprising the ligand coupled to the matrix, and a method for purification of sample proteins utilizing the chromatography matrix. Furthermore, the present disclosure relates to a nucleic acid encoding the recombinant polypeptide and to an expression system comprising the nucleic acid.

## Description

### TECHNICAL FIELD

The present disclosure generally relates to a recombinant polypeptide comprising an IgG-binding domain derived from *Streptococcus* Protein G (SpG) and a metal-binding motif. The present disclosure further relates to a ligand comprising the recombinant polypeptide, a chromatography matrix comprising the ligand coupled to the matrix, and a method for purification of sample proteins utilizing the chromatography matrix. The present disclosure also relates to a nucleic acid encoding the recombinant polypeptide and to an expression system comprising the nucleic acid.

### BACKGROUND

Antibodies, also known as immunoglobulins (Ig), are commonly used in the field of biological sciences, and represent an important class of biotherapeutic drugs. Antibodies may be used to treat various diseases, including allergy, autoimmune diseases, and cancer. An antibody is a relatively large, Y-shaped protein, and contains two antigen-binding fragments (Fab), and one crystallizable fragment (Fc) forming the trunk of the Y-shape.

Immunoglobulin G (IgG) is the main type of antibody found in blood and extracellular fluid and represents the majority of the antibody-based immunity against invading pathogens.

Bacteria such as staphylococci and streptococci have evolved to express surface proteins with an inherent affinity to immunoglobulins (Ig) with broad species specificity as a means of evading an immune response. As proven through several engineering efforts, these proteins can serve as model systems since they are well characterized in terms of structural properties, interactions, and thermodynamic stability.

Furthermore, such Ig-binding proteins are often used in affinity chromatography applications for recovery of immunoglobulins (Ig) from different samples.

*Staphylococcus aureus* protein A (SpA) is a widely used affinity chromatography ligand. SpA binds with high affinity to the Fc region of immunoglobulins.

To date, various efforts have been made to increase the binding capacity of protein A and domains thereof through recombinant technologies. Furthermore, several methods have been developed to render protein A less susceptible to alkaline pH, which is commonly used in the elution step in affinity chromatography.

EP 3 523 318 B1 relates to an IgG-binding polypeptide mutant of domain Z derived from SpA domain B, which binds to the Fc-region of an immunoglobulin, and which comprises a metal-binding motif inserted between helix 2 and 3 of the Z domain. The IgG-binding polypeptide mutant allows for elution of IgG by removal of calcium at a pH of 5.5 to 7.0.

Streptococcal protein G (SPG) is another protein with affinity to immunoglobulins. SPG generally binds to the Fc fragment of IgGs from various mammalian species.

SPG consists of repetitively arranged domains, wherein the C-terminal domains (C1, C2 and C3) are responsible for IgG-binding, and the N-terminal domains bind to human serum albumin.

The tertiary structure as well as the amino acid composition of streptococcal protein G (SPG) differs significantly from the tertiary structure and amino acid composition of protein A. Hence, these proteins have different binding characteristics. SPG may e.g. be used for the purification of IgGs, which do not bind well to protein A.

As the market for therapeutic antibodies is rapidly growing, the demand for new and improved ligands is also increasing. Hence, IgG-binding polypeptides derived from streptococcal protein G (SPG) will become important for recovery and isolation of immunoglobulins or fragments thereof. There is consequently a need to provide an improved SPG which binds selectively and with high affinity to immunoglobulins (IgG), and which may be used to recover and isolate IgGs from target samples.

### SUMMARY

In view of the above-mentioned problems, it is an object of the present disclosure to provide improvements with respect to IgG-binding polypeptides derived from streptococcal protein G.

According to a first aspect of the present disclosure, there is provided a recombinant polypeptide comprising an IgG-binding domain derived from *Streptococcus* Protein G (SPG) and a metal-binding motif, wherein the recombinant polypeptide has a first, target-binding configuration in the presence of metal ions, and a second, non-target-binding configuration in the absence of metal ions, wherein the recombinant polypeptide is configured to bind to IgG in the target-binding configuration.

The inventors have found that a metal-binding motif may be successfully introduced in an IgG-binding domain derived from SPG and have thereby provided a recombinant metal-dependent protein G-binding domain.

Upon binding of a metal ion, the recombinant polypeptide can switch from an inactive state; i.e. non-target binding configuration, to a functional, i.e. target-binding configuration.

The inventors have made an engineering effort and combined a semi-rational protein design and selection by *Escherichia coli* display followed by fluorescence-activated cell sorting and isolated several polypeptides where the inherent target affinity to antibodies and antibody fragments have been rendered metal ion dependent. Once depleted of metal ions, the target interaction of these proteins is lost.

Introduction of a metal-binding motif into a naturally occurring antibody-binding protein domain combined with directed evolution using bacterial display resulted in several recombinant polypeptides comprising switch-like binding properties with intact affinity to IgG solely in the presence of metal ions.

As used herein, the term "recombinant polypeptide" means a polypeptide formed by using recombinant DNA techniques. The recombinant polypeptide is derived from an IgG-binding domain of streptococcal protein G (SPG), into which a metal-binding motif is introduced. The recombinant polypeptide may also be referred to as an engineered polypeptide.

The term "IgG-binding domain" means the C1, C2 or C3 domain of *Streptococcus* protein G (SPG).

The IgG-binding domain; i.e. the C1, C2 or C3 domain may be intact, but the introduction of the metal-binding motif into the domain provides a recombinant polypeptide. It is also conceivable that the IgG-binding domain comprises one or a plurality of mutations.

The "metal-binding motif' is typically a metal-binding loop. A loop is a peptide fragment generally void of secondary structure and which may impact protein folding. The metal-binding loop binds metal ions, and in doing so, the loop (and the recombinant polypeptide) switches configuration from an inactive, non-target binding configuration to a configuration in which the polypeptide binds to an immunoglobulin (IgG).

The recombinant polypeptide may have a different conformation in the first, target-binding configuration, and the second, non-target-binding configuration. For example, the folding of the metal-binding motif; i.e. metal-binding loop may be different.

As mentioned hereinbefore, the IgG-binding domains of streptococcal protein G include domain C1, C2, and C3. Accordingly, the recombinant polypeptide of the present disclosure may comprise domain C1, C2 or C3.

The C domains of streptococcal protein G; i.e. domain C1, C2, and C3 are small (55 residue) peptides with immunoglobulin-binding activity.

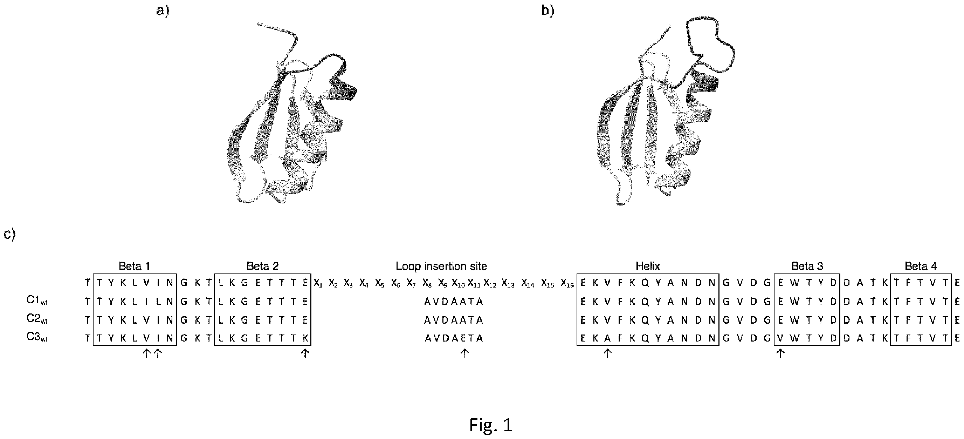

Domain C1, C2 and C3 of streptococcal protein G each consists of four antiparallel beta-sheets crossed over by an alpha helix. The C domains have a similar structure but differ slightly in the amino acid sequences. Figure 1a illustrates the crystal structure of the C2 domain, wherein the insertion site for the metal-binding loop is marked in darker grey. In figure 1b, an exemplary metal-binding loop (C2_{CaV3}) has been inserted.

The metal-binding motif is typically inserted N-terminally (upstream) of the alpha helix of the IgG-binding domain, i.e. domain C1, C2 or C3.

Preferably, the metal-binding motif is inserted between the second beta strand and the alpha helix of the IgG-binding domain, i.e. domain C1, C2 or C3 (see figure 1c).

Figure 1c illustrates the sequences of domain C1, C2, and C3, respectively (wild type), and the metal-binding loop insertion site. Domain C1 differs from domain C2 in two positions located in the first beta sheet (see arrows in figure 1c). Domain C3 differs from domain C2 in three positions throughout the scaffold, one in the second beta sheet, one in the alpha helix, and one in the third beta sheet (see arrows in figure 1c).

Typically, the IgG-binding domain is domain C1 or domain C2.

In exemplary embodiments, the recombinant polypeptide is configured to bind to the Fab region of the immunoglobulin (IgG) in the first, target-binding configuration.

Hence, the binding between the recombinant polypeptide and the IgG is highly selective and specific.

According to published crystal structures (PDB: 1FCC (1) and 1IGC (2)), the binding surface for Fab is mainly located to the second beta-sheet and the C-terminal end of the alpha helix.

As mentioned hereinbefore, the IgG-binding domains of protein G predominantly interact with the Fc region of IgG. The inventors have surprisingly found that the recombinant polypeptides of the present disclosure can selectively bind to the Fab-region of IgG with high specificity.

The recombinant polypeptides of the present disclosure may thus be used to isolate Fab fragments. Beyond therapeutic applicability, Fab fragments may be used for co-crystallization experiments to aid in protein structure determination and for visualization of tumors through radio imaging (3).

The metal-binding motif may be a calcium-binding motif, and wherein the metal ions are calcium ions.

The metal-binding motif is preferably a metal-binding loop comprising 10-14 amino acids, preferably from 12-13 amino acids.

Such a metal-binding loop is stable, and the loop structure is maintained intact. If the metal-binding loop is too small or too big, the stability and structure of the loop may be impaired. If the loop is too small, the folding of the loop, and the ability to bind metal-ions may be impaired.

The metal-binding motif may be defined by the following sequence:
X₄X₅X₆X₇X₈X₉X₁₀X₁₁X₁₂X₁₃X₁₄X₁₅X₁₆ (SEQ ID NO: 1), wherein
X₄ is aspartic acid (D),
X₅ is alanine (A), arginine (R), glutamic acid (E), lysine (K), serine (S), threonine (T), leucine (L), or valine (V),
X₆ is aspartic acid (D), or asparagine (N),
X₇ is arginine (R), asparagine (N), glycine (G), or lysine (K),
X₈ is asparagine (N), aspartic acid (D), or serine (S), preferably aspartic acid (D),
X₉ is glycine (G),
X₁₀ is arginine (R), glycine (G), lysine (K), phenylalanine (F), threonine (T), histidine (H), or tyrosine (Y), preferably phenylalanine (F), tyrosine (Y) or arginine (R),
X₁₁ is isoleucine (I), leucine (L), or valine (V),
X₁₂ is asparagine (N), aspartic acid (D), glutamic acid (E), serine (S), or threonine (T), preferably asparagine (N) or aspartic acid (D),
X₁₃ is alanine (A), phenylalanine (F), threonine (T), or tyrosine (Y),
X₁₄ is aspartic acid (D), glutamic acid (E), or lysine (K)
X₁₅ is aspartic acid (D), or glutamic acid (E), preferably aspartic acid (D),
X₁₆ is alanine (A), leucine (L), phenylalanine (F), or valine (V).

Preferably, the metal-binding motif comprises a plurality of polar amino acids.

The plurality of polar amino acids may be selected from arginine (R), asparagine (N), glutamine (Q), glutamic acid (E), histidine (H), lysine (K), serine (S), and threonine (T). Polar side chains contain groups that are either charged at pH 7 (e.g. aspartic acid and glutamic acid) or groups that are able to participate in hydrogen bonding.

For example, the metal-binding motif may comprise a polar amino acid residue in position X₄, X₆, X₇, X₈, X₁₀, X₁₂, X₁₄, and X₁₅.

Preferably, at least some of polar amino acids are negatively charged. The polar and negatively charged amino acids are believed to coordinate calcium by pointing inwards to the loop.

Preferably, the metal-binding motif comprises at least three, preferably four amino acids selected from aspartic acid (D) or asparagine (N).

Preferably, the metal-binding motif contains aspartic acid (D) in position X₄, position X₈, and position X₁₅. The metal-binding motif may also contain aspartic acid (D) in position X₁₂.

Preferably, the metal-binding motif contains phenylalanine (F), tyrosine (Y) or arginine (R) in position X₁₀.

The inventors have found that an improved binding performance with less protein ending up in the flow-through is obtained if X₁₀ is phenylalanine (F), tyrosine (Y) or arginine (R).

The recombinant polypeptide may further comprise a linker upstream of the metal-binding motif, wherein the linker comprises 1 to 4 amino acids, preferably 2 to 3 amino acids.

The linker prevents the protein structure from becoming disrupted. The linker is arranged N-terminally of the metal-binding motif, i.e. the metal-binding loop.

The linker preferably comprises 2 or 3 amino acids selected from serine (S), threonine (T), glycine (G) and/or proline (P).

Serine and threonine impart polar characteristics, whereas glycine is associated with flexibility. Proline imparts rigidity to the metal-binding motif.

In exemplary embodiments, the linker contains at least one proline (P) residue. The metal-binding motif comprising the linker may be defined by:
X₁X₂X₃X₄X₅X₆X₇X₈X₉X₁₀X₁₁X₁₂X₁₃X₁₄X₁₅X₁₆ (SEQ ID NO: 2), wherein
X₁ is serine (S), threonine (T), glycine (G), proline (P), or absent,
X₂ and X₃ are selected from serine (S), threonine (T), glycine (G) or proline (P).

In SEQ ID NO:2 hereinabove, X₁X₂X₃ represents the linker.

The recombinant polypeptide of the present disclosure may be defined by:
TTYKLILNGKTLKGETTTEX₁X₂X₃X₄X₅X₆X₇X₈X₉X₁₀X₁₁X₁₂X₁₃X₁₄X₁₅X₁₆EKVFKQYAN DNGVDGEWTYDDATKTFTVTE (SEQ ID NO:3);
TTYKLVINGKTLKGETTTEX₁X₂X₃X₄X₅X₆X₇X₈X₉X₁₀X₁₁X₁₂X₁₃X₁₄X₁₅X₁₆EKVFKQYA NDNGVDGEWTYDDATKTFTVTE (SEQ ID NO:4); or
TTYKLVINGKTLKGETTTKX₁X₂X₃X₄X₅X₆X₇X₈X₉X₁₀X₁₁X₁₂X₁₃X₁₄X₁₅X₁₆EKAFKQYA NDNGVDGVWTYDDATKTFTVTE (SEQ ID NO:5), or
a corresponding sequence having at least 80%, preferably at least 90 %, more preferably at least 95% sequence homology with the sequence.

SEQ ID NO:3 represents a recombinant polypeptide comprising domain C1.

SEQ ID NO:4 represents a recombinant polypeptide comprising domain C2.

SEQ ID NO:5 represents a recombinant polypeptide comprising domain C3.

The metal-binding motif by a sequence selected from SEQ ID NO: 6-19 or a corresponding sequence having at least 80%, preferably at least 90 %, more preferably at least 95% sequence homology with the sequence.

Accordingly, the metal-binding motif may be selected from:
DVNNDGFIDFEDA (SEQ ID NO:6),
DKDGDGRLNYKDA (SEQ ID NO:7),
DVNRDGKINAKDA (SEQ ID NO:8),
DTNKDGFVDFKDA (SEQ ID NO:9),
DVNKDGRVDFKDA (SEQ ID NO:10),
DTNNDGYVNFKDA (SEQ ID NO:11),
DTNRDGRVDYKDA (SEQ ID NO:12),
DTNRDGKVTFEDA (SEQ ID NO:13),
DKNNDGKINYKDA (SEQ ID NO:14),
DANRDGRIDAKDA (SEQ ID NO:15),
DEDGNGYINFEDA (SEQ ID NO:16),
DANKDGKINAKDA (SEQ ID NO:17),
DANRDGRINADDA (SEQ ID NO:18),
DANKDGKINAEDA (SEQ ID NO:19), or
a corresponding sequence having at least 80%, preferably at least 90 %, more preferably at least 95% sequence homology with the sequence.

The IgG-binding domain may comprise at least one mutation, wherein the mutation may be a replacement of glutamic acid (E) with glycine (G) in the first amino acid residue of the alpha helix of the IgG-binding domain.

The inventors have found that replacing the glutamic acid residue in this position; i.e. the first amino acid residue of the alpha helix of the IgG-binding domain impacts the ability of the metal-binding motif (and the recombinant polypeptide) to retain its calcium dependence.

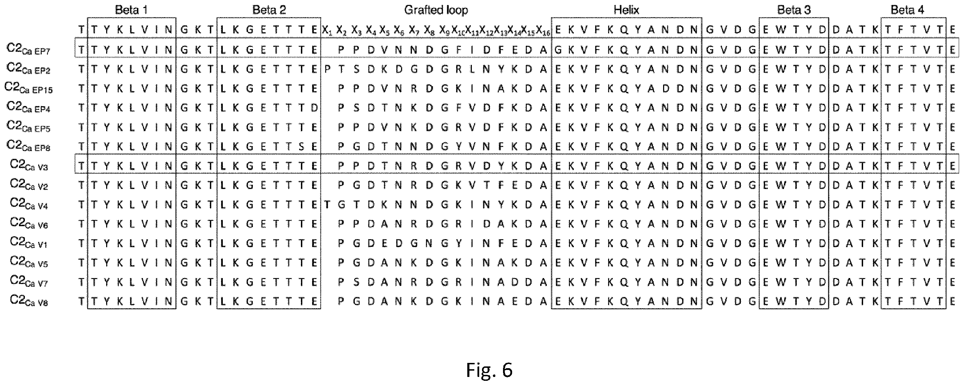

The recombinant polypeptide of the present disclosure may be defined by a sequence selected from SEQ ID NO:20-38, or a corresponding sequence having at least 80%, preferably at least 90%, more preferably at least 95% sequence homology with the sequence (see also figure 6).

According to another aspect, there is provided a ligand comprising a recombinant polypeptide as described hereinabove.

The ligand may be a multimer, e.g. a dimer, trimer, tetramer, pentamer, hexamer, heptamer, or an octamer.

The ligand may further comprise linking peptides and/or signal peptides between the monomer units. The ligand may also comprise a C-terminal cysteine for subsequent coupling to a chromatography resin of a chromatography matrix.

According to another aspect, there is provided a chromatography matrix comprising the ligand as described hereinbefore coupled to the matrix.

The chromatography matrix is not limited to a specific matrix, but any matrix known to the skilled person may be utilized. For example, the chromatography matrix may be of natural or synthetic origin. Preferably, the chromatography matrix is a solid matrix, e.g. a polysaccharide matrix.

The ligand may be coupled to the matrix by any coupling technique known to the skilled person. For example, amino acids and/or carboxy group present in the ligand may be utilized. Alternatively, the ligand may be coupled to the solid support of the matrix by thioether bonds. In this regard, the ligand may be provided with a terminal cysteine residue for subsequent use in the coupling.

Alternatively, a spacer molecule may be introduced between the matrix support and the ligand to facilitate the coupling of the ligand to the matrix.

It is also conceivable that the ligand is coupled to the matrix by non-covalent bonding, such as physical adsorption or biospecific adsorption.

According to yet another aspect, there is provided a method for purification of sample proteins comprising:
a) providing a chromatography matrix as described hereinbefore,
b) adding metal ions to the chromatography matrix,
c) adding a sample comprising target proteins,
d) eluting bound target proteins, preferably at a pH from 5 to 8.

The sample comprising target proteins may be antibodies or parts thereof, such as IgG or Fab-fusion proteins. The antibodies or parts thereof may be present in complex sample mixtures, e.g. culture supernatants.

The step d) of eluting bound target proteins may be carried out at a pH from 5 to 8, preferably from 5.5 to 7.

The step d) of eluting the bound target proteins typically comprises adding an agent which disrupts the bond between the metal ions and the metal-binding motif. For example, the agent may be a metal-binding molecule, a salt, such as sodium chloride (NaCl) and/or a chelating agent, e.g. EDTA, EGTA, glutamate diacetate or citrate glutamate.

According to another aspect, there is provided a nucleic acid encoding a recombinant polypeptide as described hereinabove.

Hence, the present disclosure also encompasses a DNA sequence that can be used in the production of the recombinant polypeptides by expression thereof in a recombinant host according to well-established biotechnological methods.

The DNA sequence may be defined by SEQ ID NO:39 or a corresponding sequence having at least 90 %, preferably at least 95%, more preferably at least 99% sequence homology with this sequence.

SEQ ID NO:39 encodes the recombinant polypeptide defined by SEQ ID NO:20.

Hence, according to a further aspect, there is provided an expression system comprising a nucleic acid as described hereinabove.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1a illustrates the crystal structure of the C2 domain of streptococcal protein G (SPG), wherein the grafting site for insertion of a metal-binding loop is shown in dark grey.
Figure 1b illustrates the crystal structure of a recombinant polypeptide according to an exemplary embodiment of the present disclose. The metal-binding loop insertion site is shown in dark grey.
Figure 1c illustrates the sequences of the wild type domains C1, C2 and C3, as well as the sequence of the linker and the metal-binding motif inserted between the second beta strand and the alpha helix of the C2 domain.

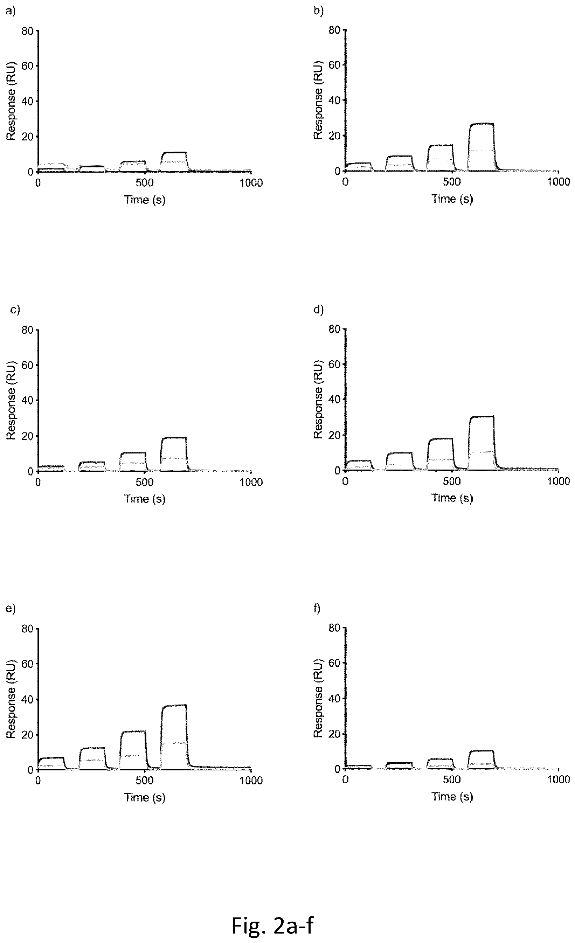

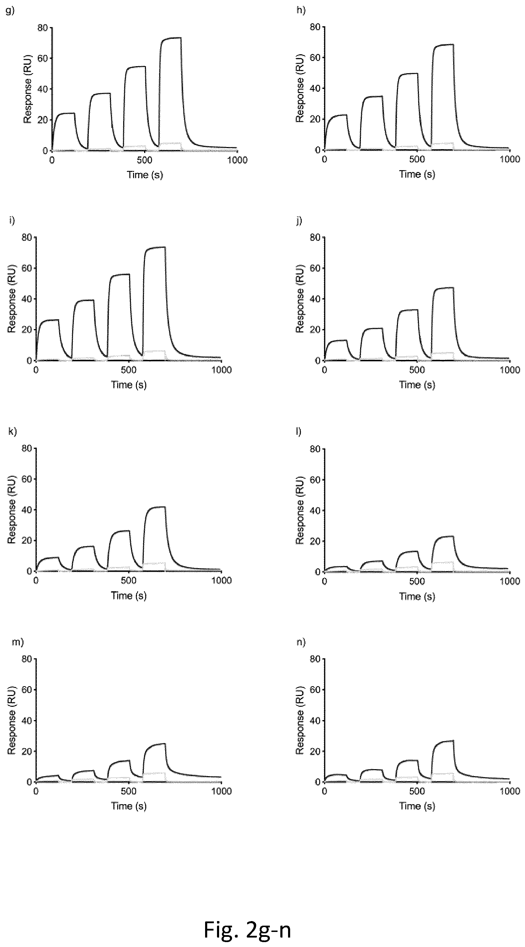

Figures 2a-n illustrates the target interaction, measured by surface plasmon resonance (SPR), in the presence (black) and absence (grey) of calcium for a plurality of recombinant polypeptides according to exemplary embodiments of the present disclosure; i.e. C2_{CaEP7} (SEQ ID NO:20) (Fig. 2a), C2_{CaEP2} (SEQ ID NO:26) (Fig. 2b), C2_{CaEP15} (SEQ ID NO:27) (Fig. 2c), C2_{CaEP4} (SEQ ID NO:28) (Fig. 2d), C2_{CaEP5} (SEQ ID NO:29) (Fig. 2e), C2_{CaEP8} (SEQ ID NO:30) (Fig. 2f), C2_{CaV3} (SEQ ID NO:31) (Fig. 2g), C2_{CaV2} (SEQ ID NO: 32) (Fig. 2h), C2cav4 (SEQ ID NO:33) (Fig. 2i), C2_{CaV6} (SEQ ID NO:34) (Fig. 2j), C2_{CaV1} (SEQ ID NO:35) (Fig. 2k), C2_{CaV5}(SEQ ID NO:36) (Fig. 2l), C2_{CaV7} (SEQ ID NO:37) (Fig. 2m), and C2_{CaV8} (SEQ ID NO:38) (Fig. 2n).

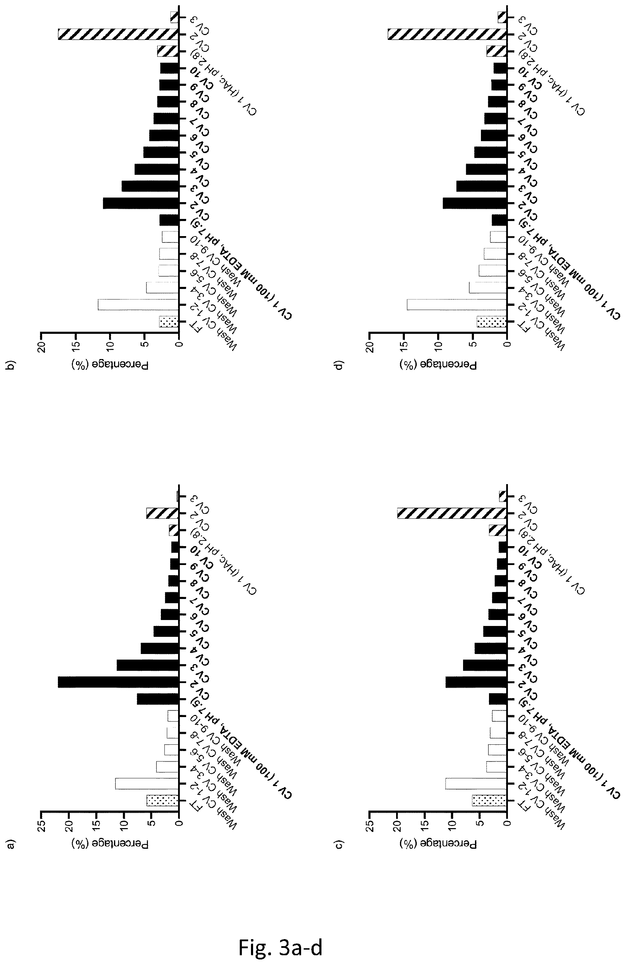

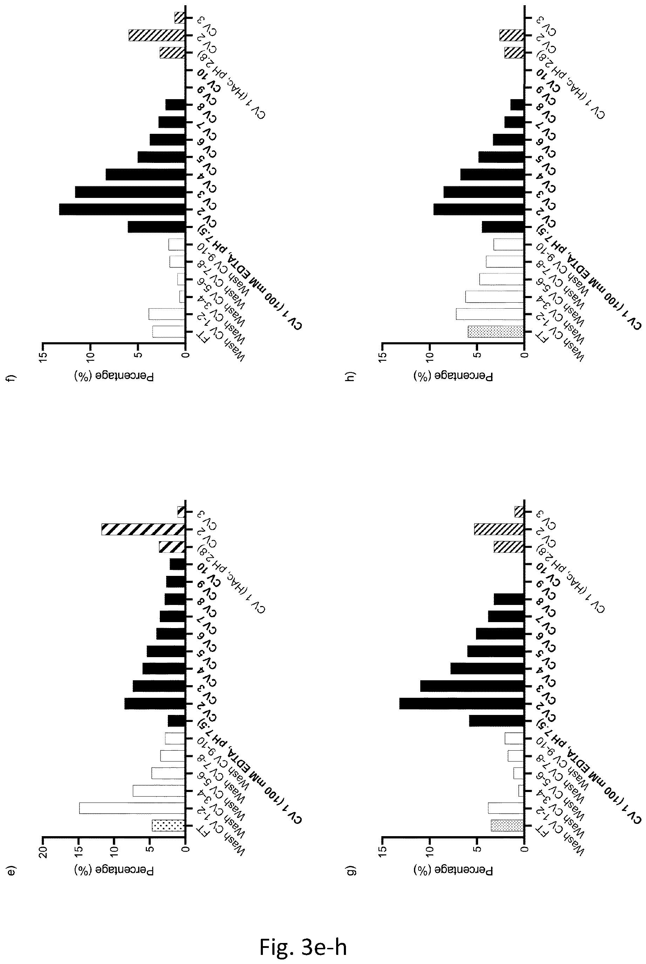

Figures 3a-h illustrate the elution profiles for a plurality of recombinant polypeptides according to exemplary embodiments of the present disclosure; i.e. C2_{CaEP7} (SEQ ID NO:20) (Fig. 3a), C2_{CaEP2} (SEQ ID NO:26) (Fig. 3b), C2_{CaEP15} (SEQ ID NO:27) (Fig. 3c), C2_{CaEP4} (SEQ ID NO:28) (Fig. 3d), C2_{CaEP5} (SEQ ID NO:29) (Fig. 3e), C2_{CaEP7_F28R} (SEQ ID NO:21) (Fig. 3f), C2_{CaEP7_F28Y} (SEQ ID NO:22) (Fig. 3g), C2_{CaEP7_F28H} (SEQ ID NO:23) (Fig. 3h) when applying 0.5 mg of each variant onto a 0.5 ml IgG sepharose column under neutral pH (7.5) conditions with elution by addition of 100 mM chelator followed by an acidic control elution using 0.5 M Hac, pH 2.8.

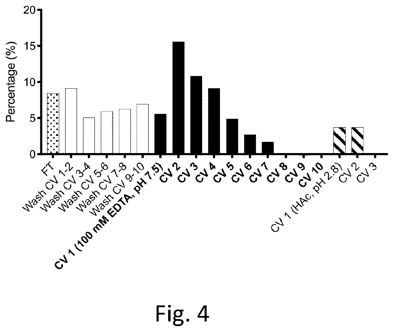

Figure 4 illustrates the elution profile for a recombinant polypeptide according to an exemplary embodiment of the present disclosure; i.e. C1_{CaEP7} (SEQ ID NO:25) when applying 0.5 mg of each variant onto a 0.5 ml IgG sepharose column under neutral pH (7.5) conditions with elution by addition of 100 mM chelator followed by an acidic control elution using 0.5 M Hac, pH 2.8.

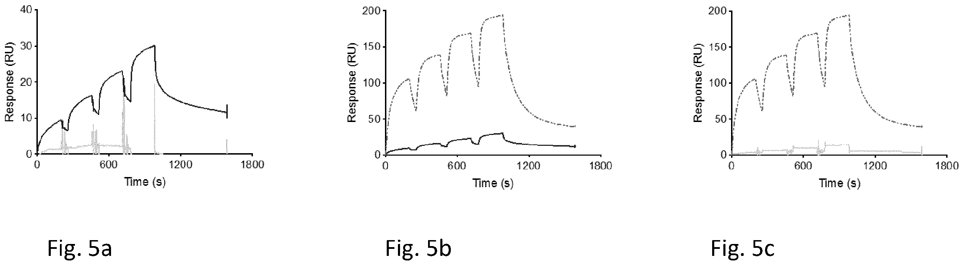

Figures 5a-c illustrate SPR sensorgrams of monomeric and tetrameric C2_{CaEP7} showing a retained calcium-dependent interaction with mIgG1 at pH 7.5 and improved binding capacity when multimerizing. Figure 5a represents the binding analysis of the interaction between C2_{CaEP7} (SEQ ID NO:20) and full-length mIgG1 during a concentration series ranging from 125 nM-1000 nM in the presence (black) and absence (grey) of calcium. Figure 5b illustrates the immobilization of equimolar amounts of monomeric (solid line) and tetrameric (dashed line) C2_{CaEP7} with the same injection series of mIgG1 showing a higher obtained binding capacity for the multimer. Figure 5c illustrates the tetrameric version of C2_{CaEP7} in the presence (dashed line) and absence (grey) of calcium, displaying the same calcium-dependency as the monomer when allowed to interact with injected mIgG1 (125 nM-1000 nM).
Figure 6 illustrates sequences of the recombinant polypeptide according to exemplary embodiments of the present disclosure, which are able to switch between a target-binding configuration in the presence of calcium ions, and a non-target binding configuration in the absence of calcium ions.

### DETAILED DESCRIPTION

The present invention provides novel recombinant polypeptides derived from the IgG-binding domains C 1, C2, and C3 of streptococcal protein G (SPG). The target binding of the IgG-binding domains has been rendered metal ion dependent by the introduction of a metal-binding motif. Accordingly, the recombinant polypeptides display a switchable target affinity depending on the presence of metal ions.

The recombinant polypeptides may be used as ligands for purification of immunoglobulins (Ig) by affinity chromatography. Such ligands may circumvent the currently harsh elution conditions associated with immunoglobulin G purification.

Thus, the invention provides a modular way of tailoring the target affinity of several protein domains with inherent antibody affinity and new tools optimizable for gentle IgG purification, particularly Fab-fragment purification.

According to a first aspect of the present disclosure, there is provided a recombinant polypeptide comprising an IgG-binding domain derived from *Streptococcus* Protein G (SPG) and a metal-binding motif, wherein the recombinant polypeptide has a first, target-binding configuration in the presence of metal ions, and a second, non-target-binding configuration in the absence of metal ions, wherein the recombinant polypeptide is configured to bind to the Fab-region of IgG in the target-binding configuration.

According to a second aspect, there is provided a ligand comprising the recombinant polypeptide described hereinbefore.

According to a third aspect, there is provided a chromatography matrix comprising the ligand coupled to the matrix.

According to a fourth aspect, there is provided a method for purification of sample proteins.

According to a fifth aspect, there is provided a nucleic acid encoding a recombinant polypeptide as defined hereinabove.

According to a sixth aspect, an expression system comprising the nucleic acid is provided.

### Experimental section

### Materials and methods

### Library creation, first generation

The library was designed to consist of the C2 domain with a randomized stretch of 12 amino acids between the second beta strand and the alpha helix. Upstream of this stretch a linker region of 0-3 amino acids was included and oligonucleotides encoding this library was obtained using trinucleotide synthesis (Twist Bioscience, San Francisco, CA, US). 200 ng of DNA was PCR-amplified during 20 cycles before performing traditional cloning to insert it into the pBad2.2 *E. coli* display vector (10). The final ligation product was electroporated into BL21(DE3) cells (Lucigen, Middleton, WI, US) which were cultivated in 100 ml LB media supplemented with 100 µg/ml carbenicillin overnight at 37°C, 150 rpm. Cells were harvested through centrifugation the following morning and the practical library size was calculated to be 1.5 × 10⁸ variants through replicate titrations. Stocks containing 15% glycerol was made with 1.5 × 10⁹ cells and stored at - 80°C.

### Library creation, second generation

Four previous selection outputs originating from selection round five and six from the library described above, extracted from cultivated cells harboring the genotype with QIAprep Spin Miniprep Kit (Qiagen, Hilden, Germany) according to the supplied protocol, was obtained. The obtained plasmid pools were subjected to four consecutive rounds of error prone-PCR using 0.1 pg template and the GeneMorph II kit (Agilent, Santa Clara, CA, US). The fragments were purified and introduced into the pBad2.2 *E. coli* display vector by traditional cloning. The ligation products were electroporated into BL21(DE3) cells (Lucigen, Middleton, WI, US) which were cultivated in 100 ml LB media supplemented with 100 µg/ml carbenicillin overnight at 37°C, 150 rpm. Centrifugation was used to harvest the cells the following morning and the practical library sizes was estimated to range from 2 × 10⁶ - 9 × 10⁷ variants.

### Target preparation

Mouse IgG1 (mIgG1) and human polyclonal Fab fragment (hFab) was ordered from Jackson ImmunoResearch (West Grove, PA, US) and biotinylated using EZ-link Sulfo-NHS-LC-Biotin (Thermo Scientific, Waltham, MA, US) according to the manufacturer's instructions. The biotinylation was stopped by removing non-reacted biotin using a NAP5 desalting column (Cytiva, Uppsala, Sweden).

### Escherichia coli display selections

1.5 × 10⁹ cells were cultivated in 100 ml LB containing 100 µg/ml carbenicillin at 37°C, 150 rpm until the OD reached 0.5-0.8 and the culture was induced with 0.6% w/v L-arabinose and grown overnight at 25°C.

The induced *E. coli* cells were washed with TBSC (50 mM Tris, 150 mM NaCl, 1 mM CaCl₂, pH 7) before being resuspended in the same buffer but containing 150 nM of biotinylated mIgG1 or 500 nM of biotinylated hFab and incubated on a rotamixer at room temperature (RT) for two hours. Next, the cells were washed with TBSC, resuspended in 150 nM human serum albumin (HSA)-Alexa 647 conjugate and 2 µg/ml streptavidin conjugated with R-phycoerythrin (SAPE) (Invitrogen, Waltham, MA, US) and incubated dark on ice for 30 minutes. The cells were subsequently washed and lastly resuspended in cold TBSC before being sorted using a MoFlo Astrios EQ cell sorter (Beckman Coulter, Brea, CA, US). The sort gate was set to capture cells displaying the library (detected library expression with (HSA)-Alexa-647 conjugate, X-axis) and binding to the target (detected target binding with SAPE, Y-axis).

Sorted cells were collected in a 15 ml tube containing LB media and was incubated during rotation for one hour at 37°C before being inoculated to 150 ml of LB media supplemented with 100 µg/ml carbenicillin and cultivated overnight at 37°C, 150 rpm. Six rounds of selection in solution were performed following this procedure, round 2-6 also included a negative selection step using magnetic activated cell sorting (MACS) prior to the positive fluorescent activated cell sorting (FACS) described. MACS was implemented to remove non-calcium dependent binders by pre-incubating target (150 nM mIgG1 or 500 nM hFab) with streptavidin-coated Dynabeads^{™} MyOne^{™} Streptavidin C1 (Thermo Scientific) before allowing the cells to be incubated with the target-coated beads for 2 hours in MBSE (25 mM MES, 150 mM NaCl, 100 mM EDTA, pH 5.5). The magnetic beads were thoroughly washed, captured and the resulting supernatant was used as input for FACS.

For the second generation, a positive FACS round was performed as described prior to performing several MACS-rounds where the captured target-binding cells were eluted from the magnetic beads with MBSE, pH 7.

### Subcloning, protein production & purification

To produce and characterize promising candidates, single clone genes were amplified from the display vector into expression vector pET-45b(+) (MilliporeSigma, Burlington, MA, US) using primers to introduce an N-terminal His₆-tag and the *XhoI* and *NcoI* restriction sites. The resulting plasmids were sequence-verified through Sanger sequencing by Eurofins (Luxembourg, Luxembourg). *E. coli* BL21(DE3) was used for production and the cells were subjected to sonication preparatory to purifying the supernatant using table-top columns packed with IgG Sepharose 6 Fast Flow resin (Cytiva).

The buffers used for purification were MBSC (25 mM MES, 150 mM NaCl, 1 mM CaCl₂, pH 6) for equilibration, 5 mM NH₄Ac supplemented with 1 mM CaCl₂, pH 6 for washing and 0.5 M HAc, pH 2.8 for elution. Upon elution the samples were buffer exchanged on PD-10 Desalting columns (Cytiva) to TBSC and the final purity was analyzed using SDS-PAGE.

### Surface plasmon resonance (SPR)

The affinity to the native targets mIgG1, human polyclonal Fab- and human polyclonal Fc was determined using a Biacore T200 instrument (Cytiva). By amine coupling, each target was diluted in 10 mM NaAc, pH 4.5 and immobilized in separate flow cells on a Series S CM5-chip. Single-cycle kinetics was obtained at 25°C through injection of a two-fold dilution series (1000 nM, 500 nM, 250 nM, 125 nM) at a flow-rate of 30 µl/min. The experiment was performed both with TBSCT (TBSC, 0.05% (v/v) Tween20, pH 7) and TBSET (TBS, 100 mM EDTA, 0.05% (v/v) Tween20, pH 7) as running buffer and after each cycle the surfaces were regenerated with 10 mM HCl.

The responses obtained were double referenced against a blank flow cell and a corresponding buffer injection preceding determination of affinity constants applying a 1:1 Langmuir binding model.

### Affinity chromatography experiments

0.5 ml of IgG Sepharose 6 FF (Cytiva) was packed in a column and pulsed with 5 CV 0.5 M HAc (pH 2.8) and 20 CV 1 × TBS-C, pH 7.5 (50 mM Tris, 150 mM NaCl, 1 mM CaCl2). 0.5 mg of pure protein (each variant) was added followed by a wash with 10 CV TBS-C. 10CV of elution with 100 mM EDTA in TBS, pH 7.5, 0.5 ml fractions. After elution, a control elution with 3 CV of 0.5 M HAc, pH 2.8 was performed. All eluted fractions were measured at 280 nm.

### Results

### Design of a metal-coordinating library

The protein structure of C2 including its previously characterized antibody binding surfaces was investigated to find a suitable insertion site for a metal-coordinating loop that could render the inherent target affinity calcium-dependent. According to published crystal structures (PDB: 1FCC (1) and 1IGC (2)) the binding surface for Fab is mainly located to the second beta-sheet and the C-terminal end of the alpha helix while the interaction with Fc is identified to be present at the C-terminal end of the alpha helix, extending into the third beta-sheet. Hence, it was hypothesized that grafting of a calcium-binding loop preceding the alpha helix might infer a calcium-dependency without risking complete loss of functionality.

Measures to further prevent disrupting the protein structure were taken by including the option of a flexible linker N-terminally of the loop. Therefore, the library was designed to include four different linker lengths of 0-3 amino acids chosen to allow for different characteristics such as polarity (Ser/Thr), flexibility (Gly), and rigidity (Pro). Following the linker, the library was intended to cover a large number of possible calcium-binding loops consisting of 13 amino acids with designed randomizations in all but two positions (Figure 1).

### Enrichment of calcium-dependency throughout the selection process

Directed evolution using bacterial display was performed towards full-length mouse IgG1 and polyclonal human Fab-fragments in order to enrich for variants with calcium-dependent target interaction. The strategy consisted of alternating negative and positive selection pressures. The negative selection was performed in a calcium-free environment and aimed at eliminating variants that was still capable of interacting with the target independent of the presence of calcium. The positive selection was carried out with fluorescence-activated cell sorting to isolate variants with retained target affinity when supplemented with calcium. After six selection rounds, a target-binding cell population seemingly calcium-dependent could be seen.

### Second generation library

To further improve the binders, the selection output from round 5 and 6 was used as starting material for error-prone PCR aiming to insert an average of two to four amino acid substitutions per variant, randomly distributed across the domain.

Bacterial display was continuedly used as the selection system with alternating negative and positive selection rounds. Although, this time the designed strategy included using magnetic-activated cell sorting (MACS) also to isolate variants which interacted with the inherent target in the presence of calcium but could be eluted from the magnetic beads by removal of calcium.

### Inherent affinity for native targets evolved to be calcium-dependent

To investigate the conditional binding properties of the variants deeper, surface plasmon resonance was used. Full-length mIgG1 and digested mFab and mFc fragment of the same antibody was immobilized on separate surfaces prior to single-cycle injections consisting of four concentrations of each variant (125 nM, 250 nM, 500 nM and 1000 nM), both in the presence and absence of calcium. All interesting variants as well as one of the variants that seem to have a calcium independent binding (C2_{Ca EP6}) were included in the analysis. All variants except for C2_{Ca EP6} displayed a calcium-dependent binding at pH 7 both towards full-length mIgG1 and mFab while no binding signals could be obtained towards the mFc surface. Interestingly, the binding capacity seemed to vary between the variants with higher obtained responses both towards mIgG1 and mFab for C2_{CaEP2} (defined by SEQ ID NO:26), C2_{CaEP4} (defined by SEQ ID NO:28), C2_{CaEP5} (defined by SEQ ID NO:29), and C2_{CaEP15} (defined by SEQ ID NO:27) on par with the non-conditional variant C2_{Ca EP6} (Fig. 2).

It was further elucidated, using a similar experimental set-up, that C2_{CaV3} (defined by SEQ ID NO:31) was showing an intact calcium-dependent human polyclonal Fab-binding while interaction with human polyclonal Fc-fragments could not be detected (Fig. 2g).

Additionally, the measured affinities for C2_{CaV3} in comparison to C2_{wt} seemed to be in the same µM-range or slightly improved towards both murine and human antibodies when measuring target interaction at neutral pH 7.0.

### Evaluation of the calcium switch in a chromatography setting

When initially evaluating novel binders in the intended application, a purification set-up with neutral pH in every step from equilibrium till elution was utilized. The variant of interest was captured on an IgG Sepharose column and eluted by addition of a chelator. Here, a number of different variants were examined, both from the two selection rounds as well as additional variants to understand the importance of different amino acids within and around the calcium chelating loop. A control elution with acidic pH (2.8) was performed to evaluate residuals captured after chelator encounter (Fig. 3 and 4).

Interestingly, sequencing of C2_{Ca EP7} revealed one mutation in the scaffold located to the first position succeeding the inserted calcium-binding loop (i.e. the first amino acid residue of the alpha helix) showing a glutamate exchanged for a glycine. The importance of this evolved feature was investigated by back-mutating it to a glutamate residue in a version referred to as C2Ca EP7_G35E. When this version was tested for calcium-dependent elution in the exact same gentle purification set-up as previously, a majority of the sample remained bound to the column until the control elution with low pH. Further, the tolerance to different amino acids in position 25 and 28 was evaluated; i.e. position X₇ and X₁₀ of the metal-binding loop. These recombinant polypeptides are defined by SEQ ID NO:21 (C2Ca_{EP7_F28R}), SEQ ID NO:22 (C2Ca_{EP7_F28Y}), SEQ ID NO:23 (C2Ca_{EP7_F28H}), and SEQ ID NO:24 (C2Ca_{EP7_N25K}).

The change to a positively charged arginine or the larger amino acid residue tyrosine in position 28 (X₁₀ of the metal-binding loop) resulted in an improved binding capacity seen by less protein leaking through the column and ending up in the flow-through.

Hypothesizing that once a switch has been engineered it could be inserted into any available domains with similar prerequisites. Thus, the evolved calcium-binding loop, including subsequent position 35 (corresponding to the first amino acid of the alpha helix) of seemingly high importance, was inserted in the IgG-binding domain C1 of Protein G. Again, the mild purification procedure was evaluated with this variant (C1_{CaEP7} defined by SEQ ID NO: 25) and it was observed that the incorporated calcium-dependency worked almost equally well in the C1 domain.

### Multimerization for increased binding capacity

Candidate C2_{CaEP7} was evaluated further by multimerizing it to become a tetramer produced with a C-terminal cysteine alongside the monomeric version. Through thiol coupling, both monomer and tetramer C2_{Ca EP7} was immobilized to equimolar amounts on separate chip surfaces. A two-fold dilution series of full-length mIgG1 (125 nM- 1000 nM) was injected and the calcium-dependent binding previously eluded for the monomer could be seen again in this reversed set-up (Figure 5). The tetramer demonstrated higher binding signals, showing that a multimeric version would have improved binding capacity. Moreover, the complete calcium-dependency was still retained for the tetrameric version of C2_{CaEP7}.

### Discussion

Naturally occurring proteins have evolved to incorporate and transmit signals or regulate their level of activity through induced conformational changes in the protein structure. Inspired by this fine-tuned control of protein functionality, we made an engineering effort to equip an existing antibody-binding domain with metal-dependency using a combinatorial library displayed on bacterial cells. The library was designed to be introduced N-terminally of the alpha helix in the C2 domain. Further, this insertion site was believed to be in close enough proximity to both inherent binding surfaces of C2 thereby endowing calcium-dependent target affinity without risking destroying the delicate protein structure completely. By implementing cell display it was possible to early on observe that the protein structure of the library members seemed intact as they evidently could be expressed on cells and interact with the native target. The sequential utilization of MACS and FACS enabled directed removal of non-calcium dependent variants during the negative selection and focused enrichment of variants ensured to display target interaction in the presence of calcium.

Interestingly, sequencing of the screened variants showing promising calcium-dependent characteristics indicated a preference for a linker length of two amino acids preceding the introduced loop although the number of sequenced variants was too low to be of significant statistical importance. Furthermore, rigid (Pro) and polar (Ser/Thr) amino acids were observed in the linker and the positively charged amino acid (Lys) was dominant in position eleven. Additionally, the design of the loop with residues (Asp/Asn) expected to coordinate calcium was seen in the sequencing data and demonstrated through the structural determination. By utilizing error-prone PCR to produce a second-generation library for another selection campaign, we were able to further enhance the calcium dependency of the binders and also gain more information about the importance of the residues in and around the grafted loop. Notably, we can conclude that the glycine in position 35 is important to retain the calcium dependence.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

### References

1. A. E. Sauer-Eriksson, G. J. Kleywegt, M. Uhlen, T. A. Jones, Pii: S0969-2126(01)00157-5. Structure 3, 1-14 (2004).

2. J. P. Derrick, D. B. Wigley, The third IgG-binding domain from streptococcal protein G: An analysis by X-ray crystallography of the structure alone and in a complex with Fab. J. Mol. Biol. 243, 906-918 (1994).

3. F.L. Moffatt Jr., S.A. Gulac, A.N. Sarafini, G.N. Sfakanckis, R. Dop, D.S. Robinson, D. Fransceschi, J. Boggs, A.S. Livingstone, A thousand points of light or just a dim light bulb? Radiolabeled antibodies and colorectal cancer imaging, Cancer Investigation, 17 (1999), pp. 322-334.

## Claims

1. A recombinant polypeptide comprising an IgG-binding domain derived from *Streptococcus* Protein G (SpG) and a metal-binding motif, wherein said recombinant polypeptide has a first, target-binding configuration in the presence of metal ions, and a second, non-target-binding configuration in the absence of metal ions, wherein said recombinant polypeptide is configured to bind to IgG in said first, target-binding configuration.

2. The recombinant polypeptide according to claim 1, wherein said IgG-binding domain is domain C1 or domain C2.

3. The recombinant polypeptide according to claim 1 or claim 2, wherein said metal-binding motif is inserted N-terminally of the alpha helix of said IgG-binding domain, preferably between the second beta strand and the alpha helix of said IgG-binding domain.

4. The recombinant polypeptide according to any one of the preceding claims, wherein said recombinant polypeptide is configured to bind to the Fab-region of said IgG in said first, target-binding configuration.

5. The recombinant polypeptide according to any one of the preceding claims, wherein said metal-binding motif is a calcium-binding motif, and wherein said metal ions are calcium ions.

6. The recombinant polypeptide according to any one of the preceding claims, wherein said metal-binding motif is a metal-binding loop comprising from 10 to 14 amino acids, preferably from 12-13 amino acids.

7. The recombinant polypeptide according to any one of the preceding claims, wherein said metal-binding motif is defined by the following sequence:
X₄X₅X₆X₇X₈X₉X₁₀X₁₁X₁₂X₁₃X₁₄X₁₅X₁₆ (SEQ ID NO: 1), wherein
X₄ is aspartic acid (D),
X₅ is alanine (A), arginine (R), glutamic acid (E), lysine (K), serine (S), threonine (T), leucine (L) or valine (V),
X₆ is aspartic acid (D), or asparagine (N),
X₇ is arginine (R), asparagine (N), glycine (G), or lysine (K),
X₈ is asparagine (N), aspartic acid (D), or serine (S), preferably aspartic acid (D),
X₉ is glycine (G),
X₁₀ is arginine (R), glycine (G), lysine (K), phenylalanine (F), threonine (T), histidine (H), or tyrosine (Y), preferably phenylalanine (F), tyrosine (Y) or arginine (R),
X₁₁ is isoleucine (I), leucine (L), or valine (V),
X₁₂ is asparagine (N), aspartic acid (D), glutamic acid (E), serine (S), or threonine (T), preferably asparagine (N) or aspartic acid (D),
X₁₃ is alanine (A), phenylalanine (F), threonine (T), or tyrosine (Y),
X₁₄ is aspartic acid (D), glutamic acid (E), or lysine (K)
X₁₅ is aspartic acid (D), or glutamic acid (E), preferably aspartic acid (D),
X₁₆ is alanine (A), leucine (L), phenylalanine (F), or valine (V).

8. The recombinant polypeptide according to any one of the preceding claims, further comprising a linker upstream of said metal-binding motif, wherein said linker comprises 1 to 4 amino acids, preferably 2 to 3 amino acids.

9. The recombinant polypeptide according to claim 8 when dependent on claim 7, wherein said metal-binding motif comprising said linker is defined by:
X₁X₂X₃X₄X₅X₆X₇X₈X₉X₁₀X₁₁X₁₂X₁₃X₁₄X₁₅X₁₆ (SEQ ID NO: 2), wherein
X₁ is serine (S), threonine (T), glycine (G), proline (P), or absent,
X₂ and X₃ are selected from serine (S), threonine (T), glycine (G) or proline.

10. The recombinant polypeptide according to any one of the preceding claims, wherein said metal-binding motif is defined by a sequence selected from SEQ ID NO:6-19 or a corresponding sequence having at least 80%, preferably at least 90 %, more preferably at least 95% sequence homology with said sequence.

11. The recombinant polypeptide according to any one of the preceding claims, wherein said IgG-binding domain comprises at least one mutation, wherein said at least one mutation is a replacement of glutamic acid (E) with glycine (G) in the first amino acid residue of the alpha helix of said IgG-binding domain.

12. A ligand comprising a recombinant polypeptide according to any one of claims 1-11.

13. A chromatography matrix comprising the ligand according to claim 12 coupled to said matrix.

14. A method for purification of sample proteins comprising:
a) providing a chromatography matrix according to claim 13,
b) adding metal ions to said chromatography matrix,
c) adding a sample comprising target proteins,
d) eluting bound target proteins, preferably at a pH from 5 to 8.

15. Nucleic acid encoding a recombinant polypeptide as defined in any one of claims 1-11.

16. An expression system comprising a nucleic acid according to claim 15.
